(19) Europäisches Patentamt
European Patent Office
Office européen des brevets

(11) Numéro de publication: **0 080 957**
**B2**

(12) **NOUVEAU FASCICULE DE BREVET EUROPEEN**

(45) Date de publication du nouveau fascicule du brevet: **24.10.90**

(51) Int. Cl.$^5$: **C 07 C 69/34**, C 07 C 67/38, C 07 C 69/42, C 07 C 69/44

(21) Numéro de dépôt: **82420165.1**

(22) Date de dépôt: **26.11.82**

(54) **Procédé de préparation de diesters saturés linéaires.**

(30) Priorité: **01.12.81 FR 8122613**

(43) Date de publication de la demande:
**08.06.83 Bulletin 83/23**

(45) Mention de la délivrance du brevet:
**26.06.85 Bulletin 85/26**

(45) Mention de la decision concernant l'opposition:
**24.10.90 Bulletin 90/43**

(84) Etats contractants désignés:
**AT BE CH DE FR GB IT LI LU NL SE**

(56) Documents cités:
**EP-A-0 003 306**
**EP-A-0 010 581**
**EP-A-0 021 010**
**US-A-2 801 263**

**Bulletin of the Chemical Society of Japan, vol. 46,1973, pages 524-530**

**Bulletin of the Chemical Society of Japan. vol.42, 1969, pages 2920-2924**
**"Fette, Seifen, Anstrichmittel", 76. Jahrgg.(1974), pages 193-196**

(73) Titulaire: **RHONE-POULENC CHIMIE**
**25, quai Paul Doumer**
**F-92408 Courbevoie Cédex (FR)**

(72) Inventeur: **Jenck, Jean**
**2, rue Lakanal**
**F-69100 Villeurbanne (FR)**

(74) Mandataire: **Varnière-Grange, Monique et al**
**RHONE-POULENC INTERSERVICES Service Brevets Chimie Centre de Recherches de Saint-Fons B.P. 62**
**F-69192 Saint-Fons Cédex (FR)**

**EP 0 080 957 B2**

Courier Press, Leamington Spa, England.

## Description

La présente invention a pour objet un procédé de préparation de diesters saturés linéaires par carbonylation d'ester(s) α,β-insaturé(s), c'est-à-dire par réaction du monoxyde de carbone et d'un alcool sur lesdits esters insaturés.

La présente invention a plus spécifiquement pour objet un procédé de préparation d'adipates d'alkyles par carbonylation de pentène-2 oates d'alkyle.

Il est bien connu, d'après le "Bulletin of the Chemical Society of Japan, vol. 46, 1973, pages 526 et 527", qu'on obtient un mélange renfermant des diesters d'alkyles et notamment, un adipate d'alkyle, en faisant réagir du monoxyde de carbone et un alcool sur un pentène-3 oate d'alkyle, sous haute pression et à température élevée, en présence de cobalt carbonyle et d'une base azotée hétérocyclique et aromatique. Cependant le développement à l'échelle industrielle d'une telle technique, dont l'intérêt de principe n'est pas contesté, est largement compromis par son manque d'efficacité.

Il a maintenant été trouvé qu'il est possible de préparer, avec une efficacité accrue, des diesters linéaires de formule

$$R_3COO—CH_2—(R_1)_p—(CH_2)_2—COOR_2 \qquad (I)$$

dans laquelle

$R_1$ représente un radical alkylène, ayant au maximum 20 atomes de carbone et pouvant être substitué par 1 à 2 atomes de chlore ou groupes alcoxy renfermant au maximum 4 atomes de carbone,

$R_2$ représente un radical alkyle comportant au maximum 12 atomes de carbone éventuellement substitué par un ou deux groupes hydroxyles, un radical aralkyle ayant de 7 à 12 atomes de carbone ou un radical phényle,

p est égal à zéro ou à un,

par réaction, en phase liquide, du monoxyde de carbone et d'un alcool $R_3OH—R_3$ ayant la signification donnée pour $R_2$, $R_3$ et $R_2$ pouvant par ailleurs être identiques ou différents sur un ester insaturé en présence d'un catalyseur métallique, choisi dans le groupe constitué par le cobalt et les composés du cobalt, et d'une base azotée tertiaire, caractérisé en ce que ledit ester est un ester α,β-insaturé de formule

$$CH_3—(R_1)_p—(CH=CH—COOR_2 \qquad (II)$$

dans laquelle $R_1$ et $R_2$ ont les significations données ci-avant et en ce que la base azotée tertiaire est choisie parmi les hétérocycles azotés à 6 chainons, pouvant comporter un ou deux substituants choisis parmi les groupes alkyles ou alcoxy ayant au maximum 4 atomes de carbone, le groupe hydroxy et les atomes d'halogène, renfermant éventuellement 2 ou 3 doubles liaisons, et pouvant, par ailleurs, le cas échéant, être soudés à un noyau benzénique, sous réserve que les maillons adjacents à l'hétéroatome d'azote ne soient ni substitués, ni communs à deux cycles, dont le $pK_a$ est compris entre 4 et 7.

Selon le présent procédé on fait donc réagir du monoxyde carbone et un alcool sur un ester α,β-insaturé de formule

$$CH_3—(R_1)_p—CH=CH—COOR_2 \qquad (II)$$

dans laquelle

$R_1$ représente un radical alkylène, ayant au maximum 20 atomes de carbone et pouvant être substitué par 1 à 2 atomes de chlore ou groupes alcoxy renfermant au maximum 4 atomes de carbone,

$R_2$ représente un radical alkyle comportant au maximum 12 atomes de carbone éventuellement substitué par un ou deux groupes hydroxyles, un radical aralkyle ayant de 7 à 12 atomes de carbone ou un radical phényle, et

p est égal à zéro ou à un.

De préférence p est égal à 1; $R_1$ est avantageusement un radical $—(C_2)_n—$, n étant un entier supérieur ou égal à 1 et inférieur ou égal à 6, pouvant comporter 1 à 2 substituants méthyle; $R_2$ est plus particulièrement un radical alkyle ayant au maximum 4 atomes de carbone.

Parmi les esters, α,β-insaturés, matières de départ du présent procédé, les pentène-2 oates d'alkyles revêtent un intérêt tout particulier, car ils permettent d'accéder efficacement aux adipates d'alkyles, intermédiaires de l'acide adipique. La demanderesse a trouvé de manière tout à fait inattendue que les esters, α,σ-insaturés sont particulièrement réactifs dans la procédé en cause, tout en permettant d'accéder au diester saturé linéaire correspondant avec une sélectivité remarquable.

Le présent procédé nécessite la mise en oeuvre d'un alcool de formule $R_3CH$, $R_3$ ayant la signification donnée précédemment.

A titre d'exemples d'alcools utilisables dans le cadre du présent procédé on peut citer le méthanol,

l'éthanol, l'isopropanol, le n-propanol, le tertiobutanol, le n-hexanol, le cyclohexanol, l'éthyl-2 hexanol-1, le dodécanol-1, l'éthylène glycol, l'hexanediol-1,6, l'alcool benzylique, l'alcool phényléthylique et le phénol.

On utilise de préférence un alcanol ayant au maximum 4 atomes de carbone; le méthanol et l'éthanol conviennent bien à la mise oeuvre du présent procédé.

On peut engager l'alcool et l'ester, α,β-insaturé en quantités stoechiométriques. Cependant on utilise de préférence un excès d'alcool, dans la proportion de 1 à 10, ou encore mieux, de 2 à 5 moles d'alcool par mole d'ester α,β-insaturé.

La réaction est conduite en présence d'un catalyseur métallique choisi dans le groupe constitué par le cobalt et les composés du cobalt. N'importe quelle source de cobalt susceptible de réagir avec le monoxyde de carbone dans le milieu réactionnel pour donner in-situ des complexes carbonyles du cobalt peut être utilisée dans le cadre du présent procédé.

Les sources typiques de cobalt sont par exemple le cobalt métallique finement divisé, des sels inorganiques tels que le nitrate ou le carbonate de cobalt, des sels organiques en particulier des carboxylates. Peuvent également être employés les cobalt-carbonyles ou hydrocarbonyles; le dicobaltoctacarbonyle convient bien à la mise en oeuvre du présent procédé.

Le rapport molaire entre l'ester, α,β-insaturé et le cobalt est en général compris entre 10 et 1 000. Ce rapport est avantageusement fixé à une valeur comprise entre 20 et 300.

Le procédé selon la présente invention nécessite également la présence d'une base azotée tertiaire dont le $pK_a$ est compris entre 4 et 7 et qui est choisie parmi les hétérocycles azotés à 6 chaînons, pouvant comporter un ou deux substituants choisis parmi les groupes alkyles ou alcoxy ayant au maximum 4 atomes de carbone le groupe hydroxy et les atomes d'halogène, renfermant éventuellement 2 ou 3 doubles liaisons, et pouvant, par ailleurs, le cas échéant être soudés à un noyau benzénique, sous réserve que les maillons adjacents à l'hétéroatome d'azote ne soient ni substitués, ni communs à deux cycles.

Conviennent plus particulièrement à la mise en oeuvre du présent procédé la pyridine, la picoline-4, l'isoquinoléine, la lutidine-3,5 et la lutidine-3,4.

La quantité de base azotée tertiaire utilisée est en général telle que la rapport molaire N/Co soit compris entre 0,5 et 50. Pour une bonne mise en oeuvre de l'invention la demanderesse préconnise de fixer ce rapport à une valeur comprise entre 2 et 25.

Selon la présente invention on fait donc réagir du monoxyde de carbone et un alcool ($R_3OH$) sur un ester α,β-insaturé en présence du système catalytique défini ci-avant. La réaction est conduite en phase liquide à une température généralement supérieure à 100°C, sans qui'il soit utile de dépasser 200°C, sous une pression de monoxyde de carbone d'au moins 50 bars et pouvant atteindre 1 000 bars. On préfère opérer à une température de l'ordre de 130 à 180°C et sous une pression de monoxyde de carbone de l'ordre de 100 à 300 bars.

Bien entendu, les conditions de pression et de température optimales seront d'autant plus sévères que la matière de départ sera moins réactive, ce qui se produit, notamment, lorsque le degré de protection stérique de la double liaison augment.

On utilise du monoxyde de carbones sensiblement pur, tel qu'il se présente dans le commerce. Toutefois, la présence d'impuretés telles que du dioxyde de carbone, du méthane ou de l'azote n'est pas nuisible; la présence de traces d'hydrogène (inférieures à 1,5% en volume) tend à stabiliser le système catalytique.

Comme cela a été indiqué en téte du présent mémoire, le procédé selon la présente invention trouve une application particulière dans la synthèse d'adipates d'alkyles à partir de pentène-2 oates d'alkyles. Dans le cadre de cette synthèse il s'avère avantageux de choisir l'alcool (coréactif) qui correspond au reste alkyle de l'ester de départ, le reste alkyle ayant, de préférence, 4 atomes de carbone au maximum. De bons résultats sont obtenus au départ des couples de réactifs suivants: pentène-2 oate de méthyle et méthanol, pentène-2 oate d'éthyle et éthanol.

Bien que le biester linéaire saturé soit obtenu avec une sélectivité élevée, on observe, toutefois la formation, en faibles proportions, de diesters saturés branchés et de l'ester saturé, produit d'hydrogénation de la matière de départ.

Les exemples ci-après illustrent l'invention sans toutefois en limiter le domaine ou l'esprit.

Dans les exemples, les conventions suivantes sont utilisées.

=/Co désigne le rapport molaire de l'ester α,β-insaturé au cobalt,

RT% désigne le nombre de moles du produit considéré pour 100 moles der diesters et d'ester saturé formées,

TT (%) désigne le nombre de moles de diesters et d'ester saturé formées pour 100 moles de matière de départ chargées,

T (°C) désigne la température en degrés celsius,

A désigne l'activité exprimée en moles de diesters et d'ester saturé formées par heure et par atomegramme de cobalt,

X (%) désigne le nombre de moles de diesters pour 100 moles de produits formés,

Y (%) désigne le nombre de moles d'adipate d'alkyle pour 100 moles de produits formés,

Z (%) désigne le nombre de moles de pentanoate d'alkyle pour 100 moles de produits formés.

Dans les produits formés ne sont pas inclus les composés résultant de l'isomérisation de la double

liaison oléfinique. Les produits formés sont essentiellement les diesters et le pentanoate d'alkyle, ce dernier résultant de l'hydrogénation de l'ester de depart.

Exemple 1

Dans un autoclave de 125 ml en acier inoxydable purgé sous courant d'argon, on introduit 50 mmol de crotonate de méthyle, 102 mmol de méthanol, 1 mmol de dicobaltoctacarbonyle et 7,9 mmol d'isoquinoléine. L'autoclave est alors purgé par un un courant de monoxyde de carbone renfermant 0,7% en volume d'hydrogène puis on le porte à 160°C, sous une pression de 130 bars.

Après 2 heures de réaction à cette température, l'autoclave est refroidi et dégazé. Le mélange réactionnel est analysé par chromatographie en phase gazeuse.

Les résultats obtenus sont les suivants:

TT (%) = 77,9; (A = 9,7)

| | RT (%) |
|---|---|
| glutarate de diméthyle | 77,0 |
| méthyl-2 succinate de diméthyle et éthyl-2 malonate de diméthyle | 18,0 |
| butanoate de méthyle | 5,0 |

Exemple 2

On reproduit l'exemple 1 en remplaçant le crotonate de méthyle par un quantité équivalente d'octene-2 oate de méthyle.

Les résultats obtenus sont les suivants:

TT (%) = 37,6; (A = 4,7)

| | RT (%) |
|---|---|
| nonanedioate de diméthyle | 53,2 |
| diesters saturés branchés | 30,0 |
| octanoate de méthyle | 16,8 |

Exemple 3

Dans l'autoclave et selon le mode opératoire décrits ci-avant on réalise un essai sur une charge constituée de 80 mmol d'hexene-2 oate de méthyle, 200 mmol de méthanol, 0,68 mmol de dicobaltoctacarbonyle et de 5,5 mmol d'isoquinoléine. Les résultats obtenus en deux heures de réaction, à 160°C, sous 130 bars et en utilisant du monoxyde de carbone renfermant 1,2% en volume d'hydrogène sont les suivants:

TT (%) = 50,2; (A = 14,8)

| | RT (%) |
|---|---|
| heptanedioate de diméthyle | 74,3 |
| diesters saturés branchés | 19,7 |
| hexanoate de méthyle | 5,6 |

Exemples 4 à 8

On réalise une première d'essais selon le mode opératoire décrit précédemment en faisant réagir du monoxyde de carbone contenant 0,8% (en volume) d'hydrogène sur une charge renfermant 80 mmol de pentène-2 oate de méthyle, 200 mmol de méthanol, 0,6 mmol de dicobaltoctacarbonyle et de l'isoquinoléine. Les conditions particulières ainsi que les résultats obtenus en 2 heures de réaction à 160°C, sous 130 bars sont portés dans le tableau I ci-après.

TABLEAU I

| Ex. N° | N/Co | A | X (%) | Y (%) | Z (%) |
|---|---|---|---|---|---|
| 4 | 3,6 | 18 | 93,4 | 76,4 | 5,9 |
| 5 | 8,2 | 22 | 94,7 | 78,4 | 5,9 |
| 6 | 13,2 | 13 | 95,5 | 77,6 | 4,3 |
| 7 | 19,2 | 13 | 93,4 | 75,7 | 6,5 |
| 8 | 20,1 | 13 | 94,7 | 76,8 | 7,4 |

Essais témoins (a) à (d):

On réalise une seconde série d'essais selon le mode opératoire décrit précédemment en faisant réagir du monoxyde de carbone contenant 0,8% (en volume) d'hydrogène sur une charge renfermant 80 mmol de pentène-3 oate· de méthyle, 200 mmol de méthanol, 0,6 mmol de dicobaltoctacarbonyle et de l'isoquinoléine. Les conditions particulières ainsi que les résultats obtenus en 2 heures de réaction à 160°C sous 130 bars sont portés dans le tableau II ci-après.

TABLEAU II

| Réf. | N/Co | A | X (%) | Y (%) | Z (%) |
|---|---|---|---|---|---|
| a | 3,7 | 7,8 | 94,9 | 76,0 | 6,6 |
| b | 7,4 | 7,4 | 95,1 | 80,0 | 4,3 |
| c | 13,4 | 3,9 | 95,4 | 79,5 | 3,7 |
| d | 20,5 | 3,7 | 92,4 | 78,4 | 7,4 |

Exemples 9 à 14

—Essais témoins (e) à (j)

Dans le tableau III ci-après on a rassemblé les conditions et les résultats d'une séries d'essais réalisés selon le mode opératoire décrit précédemment. Dans tous les essais on a utilisé de l'isoquinoléine, du méthanol, du dicobaltoctacarbonyle et du monoxyde de carbone renfermant 0,8% (en volume) d'hydrogène. La durée réaction a été de 2 heures. Dans les exemples 8 à 13 on a utilisé comme matière de départ le pentène-2 oate de méthyle; les essais témoins (a) à (j) sont réalisés au départ de pentène-3 oate de méthyle.

Dans le tableau III, P désigne la pression à la température de l'essai.

TABLEAU III

| Ex. n° | $CH_3OH$ mmol | $Co_2(CO)_8$ mmol | =/Co | N/Co | T°G | P bars | TT (%) | X (%) | Y (%) |
|---|---|---|---|---|---|---|---|---|---|
| 9 | 98 | 0,99 | 25,2 | 4,0 | 160 | 90 | 60,9 | 90,1 | 75,9 |
| e | 104 | 1,01 | 25,1 | 4,2 | 160 | 90 | 33,5 | 89,3 | 75 |
| 10 | 100 | 0,93 | 26,7 | 4,3 | 140 | 130 | 66,0 | 94,5 | 69,6 |
| f | 100 | 1,03 | 24,0 | 3,8 | 140 | 130 | 28,1 | 94,0 | 70,3 |
| 11 | 148 | 0,41 | 122 | 3,7 | 160 | 130 | 23,7 | 95,7 | 78,6 |
| g | 148 | 0,42 | 120 | 3,9 | 160 | 130 | 12,9 | 94,2 | 77,7 |
| 12 | 102 | 1,03 | 24 | 19,4 | 160 | 130 | 34,9 | 85,2 | 67,2 |
| h | 104 | 0.98 | 25 | 20,6 | 160 | 130 | 9,0 | 90,1 | 79,7 |
| 13 | 202 | 0,2 | 240 | 19,6 | 180 | 280 | 11,0 | 90,8 | 77,6 |
| i | 199 | 0,2 | 240 | 20,3 | 180 | 280 | 1,4 | 90,5 | 71,5 |
| 14 | 200 | 0,5 | 100 | 4 | 180 | 250 | 54,5 | 93,7 | 74,5 |
| j | 198 | 0,495 | 101 | 4,1 | 180 | 250 | 32,6 | 93,3 | 75,7 |

Exemple 15

Dans un réacteur de 300 ml acier inoxydable muni d'une turbine centrale d'agitation, chauffé et régulé électriquement on introduit un mélange constitué de 482 mmol de pentène-2 oate de méthyle, 1 050 mmol de méthanol, 3,45 mmol de dicobaltoctacarbonyle et de 27,6 mmol d'isoquinoléïne. On chauffe sous balayage de monoxyde de carbone renfermant 0,8% en volume d'hydrogène jusqu'à 160°C. La pression dans l'autoclave est maintenue à 130 bars et le débit d'alimentation du mélange gazeux est de 40 l/h (CNTP). On effectue, périodiquement, des prélèvements de la masse réactionnelle que l'on analyse.

Les résultats obtenus sont portés dans le tableau IV ci-après dans lequel la durée en heure indique l'intervalle de temps écoulé entre la mise en température (160°C) et la prise de l'échantillon considéré.

On a également indiqué dans la colonne intitulée "témoin k" la valeur des divers TT (%) correspondants, obtenus lors d'un autre essai réalisé comme l'exemple 15 mais sur un mélange constitué de 700 mmol de pentène-3 oate de méthyle, 1 525 mmol de méthanol, 40 mmol d'isoquinoléïne et 5 mmol de dicobaltoctacarbonyle. Voir Tableau IV.

TABLEAU IV

| Prélèvement | Durée | Exemple 15 | | | Témoin k |
|---|---|---|---|---|---|
| n° | (h) | X (%) | Y (%) | TT (%) | TT (%) |
| 1 | 0,5 | 95,9 | 79,3 | 18,6 | 6,3 |
| 2 | 1 | 95,2 | 78,0 | 34,5 | 13,1 |
| 3 | 2 | 93,7 | 75,3 | 58,4 | 28 |
| 4 | 3 | 93,7 | 75,2 | 75,2 | 45 |
| 5 | 4 | 93,3 | 74,2 | 88,3 | 60,7 |

**Revendications**

1. Procédé selon de préparation de diesters saturés linéaires de formule

$$R_3COO—CH_2—(R_1)_p—(CH_2)_2—COOR_2 \quad (I)$$

dans laquelle

$R_1$ représente un radical alkylène, ayant au maximum 20 atomes de carbone et pouvant être substitué par 1 à 2 atomes de chlore ou groupes alcoxy renfermant au maximum 4 atomes de carbone,

$R_2$ représente un radical alkyle comportant au maximum 12 atomes de carbone éventuellement. substitué par un ou deux groupes hydroxyles, un radical aralkyle ayant de 7 à 12 atomes de carbone ou un radical phényle,

p est égal à zéro ou à un,

par réaction, en phase liquide, du monoxyde de carbone et d'un alcool $R_3CH—R_3$ ayant la signification donnée pour $R_2$, $R_3$ et $R_2$ pouvant par ailleurs être identiques ou différents sur un ester insaturé en présence d'un catalyseur métallique, choisi dans le groupe constitué par le cobalt et les composés du cobalt, et d'une base azotée tertiaire, caractérisé en ce que le dit ester est un ester α,β-insaturé de formule

$$CH_3—(R_1)_p—CH{=}CH—COOR_2 \quad (II)$$

dans laquelle $R_1$ et $R_2$ ont les significations données ci-avant et en ce que la base azotée tertiaire est choisie parmi les hétérocycles azotés à 6 chainons, pouvant comporter un ou deux substituants choisis parmi les groupes alkyles ou alcoxy ayant au maximum 4 atomes de carbone, le groupe hydroxy et les atomes d'halogène, renfermant éventuellement 2 ou 3 doubles liaisons, et pouvant, par ailleurs, le cas échéant, être soudés à un noyau benzénique, sous réserve que les maillons adjacents à l'hétéroatome d'azote ne soient ni substitués, ni communs à deux cycles, dont le $pK_a$ est compris entre 4 et 7.

2. Procédé selon la revendication 1, caractérisé en ce que la température de réaction est comprise entre 100 et 200°C et, de préférence entre 130 et 180°C.

3. Procédé selon la revendication 1 ou 2, caractérisé en ce que la pression est comprise entre 50 et 1 000 bars et, de préférence entre 100 et 300 bars.

4. Procédé selon l'une quelconque des revendications précédentes, caractérisé en ce que l'ester α,β-insaturé est choisi parmi les composés de formule (II) dans laquelle $R_1$ est un radical $—(CH_2)_n—$, nétant un entier supérieur ou égal à 1 et inférieur ou égal à 6, pouvant comporter 1 ou 2 substituants méthyle et $R_2$ a la signification donnée dans la revendication 1.

5. Procédé selon l'une quelconque des revendications précédentes, caractérisé en ce que l'ester α,β-insaturé est choisi parmi les composés de formule (II) dans laquelle $R_1$ la signification donnée dans la revendication 1 ou 4 et $R_2$ est un radical alkyle ayant au maximum 4 atomes de carbone.

6. Procédé selon l'une quelconque des revendications précédentes, caractérisé en ce que l'ester α,β-insaturé est choisi parmi les pentène-2 oates d'alkyles dont le groupe alkyle renferme au maximum 4 atomes de carbone.

7. Procédé selon l'une quelconque des revendications précédentes, caractérisé en ce que le rapport molaire entre l'ester α,β-insaturé et le cobalt est compris entre 10 et 1 000 et, de préférence entre 20 et 300.

8. Procédé selon l'une quelconque des revendications précédentes, caractérisé en ce que le rapport molaire entre l'alcool et l'ester α,β-insaturé est compris entre 1 et 10 et de préférence entre 2 et 5.

9. Procédé selon l'une quelconque des revendications précédentes, caractérisé en ce que le rapport molaire entre le base tertiaire azotée et le cobalt est compris entre 0,5 et 50 et, de préférence entre 2 et 25.

**Patentansprüche**

1. Verfahren zur Herstellung von gesättigten linearen Diestern der Formel

$$R_3COO—CH_2—(R_1)_p—(CH_2)_2—COOR_2 \quad (I)$$

in der

$R_1$ eine Alkylengruppe mit maximal 20 Kohlenstoffatomen bedeutet, die durch 1 oder 2 Chloratome oder Alkoxygruppen mit maximal 4 Kohlenstoffatomen substituiert sein kann,

$R_2$ ein Alkylgruppe mit maximal 12 Kohlenstoffatomen, die gegebenenfalls durch 1 oder 2 Hydroxylgruppen substuiert ist, eine Aralkylgruppe mit 7 bis 12 Kohlenstoffatomen oder eine Phenylgruppe bedeutet,

p 0 oder 1 ist,

durch Umsetzung in flüssiger Phase von Kohlenmonoxid und einem Alkohol $R_3OH$, wobei $R_3$ die für $R_2$ angegebene Bedeutung hat und $R_3$ und $R_2$ gleich oder verschieden sein können, mit einem ungesättigten Ester in Gegenwart eines Metallkatalysators ausgewählt aus der Gruppe bestehend aus Cobalt und

Cobaltverbindungen, und einer tertiären Stickstoffbase, dadurch gekennzeichnet, daß der Ester ein α,β-ungesättigter Ester der Formel

$$CH_3—(R_1)_p—CH=CH—COOR_2 \quad (II)$$

ist, in der $R_1$ und $R_2$ die oben angegebenen Bedeutungen haben und daß die tertiäre Stickstoffbase unter den 6-gliedrigen stickstoffhaltigen Heterocyclen ausgewählt wird, die 1 oder 2 Substituenten aufweisen können, die ausgewählt sind unter Alkyl- oder Alkoxygruppen mit maximal 4 Kohlenstoffatomen, Hydroxygruppe und Halogenatomen, und die gegebenenfalls 2 oder 3 Dopelbindungen enthalten und außerdem gegebenenfalls mit einem Benzolkern kondensiert sein können, mit der Maßgabe, daß die dem Stickstoff-Heteroatom benachbarten Ringglieder weder substituiert sind noch gleichzeitig 2 Ringen angehören, deren $pK_a$ 4 bis 7 beträgt.

2. Verfahren nach Anspruch 1, dadurch gekennzeichnet, daß die Reaktionstemperatur 100 bis 200°C, vorzugsweise 130 bis 180°C beträgt.

3. Verfahren nach Anspruch 1 oder 2, dadurch gekennzeichnet, daß der Druck 50 bis 1000 bar, vorzugsweise 100 bis 300 bar, ausmacht.

4. Verfahren nach einem der vorangehenden Ansprüche, dadurch gekennzeichnet, daß der α,β-ungesättigte Ester unter den Verbindungen der Formel (II) ausgewählt wird, bei denen $R_1$ eine Gruppe $—(CH_2)_n—$ ist, wobei n eine ganze Zahl $\geqq 1$ und $\leqq 6$ ist, die 1 oder 2 Methylsubstituenten tragen kann und $R_2$ die in Anspruch 1 angegebene Bedeutung hat.

5. Verfahren nach einem der vorangehenden Ansprüche, dadurch gekennzeichnet, daß der α,β-ungesättigte Ester unter den Verbindungen der Formel (II) ausgewählt wird, bei denen $R_1$ die in Anspruch 1 oder 4 angegebene Bedeutung hat und $R_2$ eine Alkylgruppe mit maximal 4 Kohlenstoffatomen ist.

6. Verfahren nach einem der vorangehenden Ansprüche, dadurch gekennzeichnet, daß der α,β-ungesättigte Ester unter den Alkyl-2-pentenoaten ausgewählt wird, deren Akylgruppe maximal 4 Kohlenstoffatome umfaßt.

7. Verfahren nach einem der vorangehenden Ansprüche, dadurch gekennzeichnet, daß das Molverhältnis von α,β-ungesättigtem Ester zu Cobalt 10 bis 1000, vorzugsweise 20 bis 300 ausmacht.

8. Verfahren nach einem der vorangehenden Ansprüche, dadurch gekennzeichnet, daß das Molverhältnis von Alkohol zu α,β-ungesättigtem Ester 1 bis 10, vorzugsweise 2 bis 5 ausmacht.

9. Verfahren nach einem der vorangehenden Ansprüche, dadurch gekennzeichnet, daß das Molverhältnis von tertiärer Stickstoffbase zu Cobalt 0,5 bis 50, vorzugsweise 2 bis 25 ausmacht.

**Claims**

1. Process for preparing saturated linear diesters of formula

$$R_3COO—CH_2—(R_1)_p—(CH_2)_2—COOR_2 \quad (I)$$

in which

$R_1$ represents an alkylene radical having at most 20 carbon atoms and which can be substituted with 1 to 2 chlorine atoms or alkoxy groups containing at most 4 carbon atoms,

$R_2$ represents an alkyl radical containing at most 12 carbon atoms, optionally substituted with one or two hydroxyl groups, an aralkyl radical having from 7 to 12 carbon atoms or a phenyl radical, and

p is equal to zero or one,

by the reaction, in the liquid phase, of carbon monoxide and an alcohol $R_3OH$ — $R_3$ having the meaning given for $R_2$, it being possible, moreover, for $R_3$ and $R_2$ to be identical or different — with an unsaturated ester in the presence of a metal catalyst, selected from the group consisting of cobalt and cobalt compounds, and a tertiary nitrogenous base, characterized in that the said ester is an α,β-unsaturated ester of formula

$$CH_3—(R_1)_p—CH=CH—COOR_2 \quad (II)$$

in which $R_1$ and $R_2$ have the meanings given above, and in that the tertiary nitrogenous base is selected from 6-membered nitrogenous heterocycles which can contain one or two substituents selected from alkyl or alkoxy groups having at most 4 carbon atoms, the hydroxyl group and halogen atoms, which optionally contain 2 or 3 double bonds and which can, moreover, where appropriate, be fused to a benzene ring, with the proviso that the links adjacent to the nitrogen hetero atom are neither substituted nor common to two rings, the $pK_a$ of which is between 4 and 7.

2. Process according to Claim 1, characterized in that the reaction temperature is between 100 and 200°C, and preferably between 130 and 180°C.

3. Process according to Claim 1 or 2, characterized in that the pressure is between 50 and 1,000 bars, preferably between 100 and 300 bars.

4. Process according to any one of the preceding claims, characterized in that the α,β-unsaturated ester is selected from the compounds of formula (II) in which $R_1$ is a radical $-(CH_2)_n$, n being an integer greater than or equal to 1 and less than or equal to 6, capable of containing 1 or 2 methyl substituents, and $R_2$ has the meaning given in Claim 1.

5. Process according to any one of the preceding claims, characterized in that the α,β-unsaturated ester is selected from the compounds of formula (II) in which $R_1$ has the meaning given in Claim 1 or 4 and $R_2$ is an alkyl radical having at most 4 carbon atoms.

6. Process according to any one of the preceding claims, characterized in that the α,β-unsaturated ester is selected from alkyl 2-pentenoates in which the alkyl group contains at most 4 carbon atoms.

7. Process according to any one of the preceding claims, characterized in that the α,β-unsaturated ester to cobalt is between 10 and 1,000, and preferably between 20 and 300.

8. Process according to any one of the preceding claims, characterized in that the mole ratio of the alcohol to the α,β-unsaturated ester is between 1 and 10, and preferably between 2 and 5.

9. Process according to any one of the preceding claims, characterized in that the mole ratio of the tertiary nitrogenous base to cobalt is between 0.5 and 50, and preferably between 2 and 25.